# EUROPEAN PATENT SPECIFICATION

(11) **EP 3 174 521 B1**
(45) Date of publication and mention of the grant of the patent: **22.08.2018**
(21) Application number: 15732162.1
(22) Date of filing: 19.06.2015
(51) Int. Cl.: A61K 9/20, A61K 47/58, A61K 9/00, A61K 31/522

(54) **PEDIATRIC CHEWABLE TABLET CONTAINING ANTIVIRAL AGENT AND METHOD FOR THE PREPARATION THEREOF**
PÄDIATRISCHE KAUTABLETTE MIT ANTIVIRALEM MITTEL UND VERFAHREN ZUR HERSTELLUNG DAVON
COMPRIMÉ À MÂCHER PÉDIATRIQUE CONTENANT UN AGENT ANTIVIRAL ET SON PROCÉDÉ DE PRÉPARATION

(30) Priority: 31.07.2014 WO PCT/EP2014/002097
(43) Date of publication of application: 07.06.2017
(73) Proprietor: Pharmathen S.A., 15351 Pallini Attikis (GR)
(72) Inventor: KARAVAS, Evangelos, GR-15351 Pallini Attikis (GR); KOUTRIS, Efthymios, GR-15351 Pallini Attikis (GR); SAMARA, Vasiliki, GR-15351 Pallini Attikis (GR); KOUTRI, Ioanna, GR-15351 Pallini Attikis (GR); KALASKANI, Anastasia, GR-15351 Pallini Attikis (GR); KALANTZI, Lida, GR-15351 Pallini Attikis (GR); KAKOURIS, Andreas, GR-15351 Pallini Attikis (GR); DIAKIDOU, Amalia, GR-15351 Pallini Attikis (GR); GOTZAMANIS, George, GR-15351 Pallini Attikis (GR); GEORGOUSIS, Zaharias, GR-15351 Pallini Attikis (GR); FOUSTERIS, Manolis, GR-15351 Pallini Attikis (GR)
(86) International application number: PCT/EP2015/001244
(87) International publication number: WO 2016/015799

(56) References cited:
- WO-A1-2007/090595
- WO-A2-2009/049648
- US-A1- 2009 124 639

## Description

### TECHNICAL FIELD OF THE INVENTION

The present invention relates to a stable pharmaceutical formulation for oral administration containing a therapeutically effective quantity of an antiviral agent, and more particularly Valaciclovir or pharmaceutical acceptable salt or derivative thereof, and a method for the preparation thereof.

### BACKGROUND OF THE INVENTION

Acyclovir is a known compound widely used for the treatment of viral infections, particularly infections caused by the herpes virus. Pharmacokinetic studies have shown that acyclovir is poorly water soluble and presents poor oral bioavailability, therefore intravenous administration is required in order to achieve high concentrations in the plasma.

Valaciclovir or L-valyl acyclovir is a prodrug of acyclovir, and has been shown to possess antiviral properties. It is used for the treatment of the same types of infections as acyclovir. More specifically, it is used for varicella zoster virus infections-herpes zoster, herpes simplex virus infections and cytomegalovirus infections.

A preferred form of Valaciclovir is the Valaciclovir hydrochloride salt. After oral administration, Valaciclovir hydrochloride is rapidly absorbed from the gastrointestinal tract and nearly completely converted to acyclovir and L-valine by first-pass intestinal and/or hepatic metabolism by enzymatic hydrolysis. It has been shown that Valaciclovir provides a high bioavailability of acyclovir, much higher than that obtained with oral acyclovir, and is equivalent to plasma levels achieved with doses of intravenous acyclovir.

The chemical name of Valaciclovir hydrochloride is L-valine, 2-[(2-amino-1, 6-dihydro-6-oxo-9H-purin-9-yl) methoxy] ethyl ester, mono hydrochloride and its molecular formula is C₁₃H₂₀N₆O₄•HCl corresponding to a molecular weight of 360.80 (for HCl salt) and 324.34 (for free base). It is a white to off-white crystalline powder. Valaciclovir hydrochloride is soluble in water and insoluble in dichloromethane, the maximum solubility in water at 25° C is 174 mg/mL.

US 5869095 B discloses a chewable tablet with an effervescent action and a tablet weight of less than 3 g, containing at least one pharmaceutically active substance, an effervescent base comprising a solid, edible, organic acid and an alkali metal and/or alkaline earth metal carbonate and/or bicarbonate and at least one soluble filler, characterized in that the acid particles and/or the carbonate particles of the effervescent base are coated with a hydrocolloid.

EP 1517617 B1 discloses a compressed chewing gum tablet comprising a chewing gum center, said gum center comprising a compression of gum base granules and chewing gum additives, said chewing gum additives comprising sweeteners and flavors, at least a first part of said gum base granules comprising flavor or active ingredients incorporated gum base, at least a second part of said gum base granules comprising granules of conventional gum base.

Although each of the patents above represents an attempt to provide a stable and patient-friendly Valaciclovir composition for oral administration, there still remains the need in the art for age-adapted dosage forms appropriate for children. In particular, there is a need for safe and effective taste masked tablet formulation ideal for children.

### SUMMARY OF THE INVENTION

It is, therefore, an object of the present invention to provide a stable solid dosage formulation for oral administration containing an antiviral agent, and in particular Valaciclovir or pharmaceutical acceptable salt or derivative thereof as an active ingredient, which is adequate for pediatric use.

The main object of the present invention is to provide a chewable tablet comprising Valaciclovir, which achieves to mask the bitter taste of the active ingredient and provides acceptable palatability.

Another object of the present invention is the selection of the optimal combination of pharmaceutical acceptable excipients and method of preparation in order to achieve the appropriate dissolution profile and stability for the finished dosage form.

Moreover, it is another object of the present invention to provide a chewable tablet comprising Valaciclovir, which can be formulated into dosage forms of different strengths by proportionally adjusting the amounts of the pharmaceutically acceptable excipients, as well as the active compound Valaciclovir.

A further approach of the present invention is to provide a chewable tablet comprising Valaciclovir or pharmaceutical acceptable salt or derivative thereof which is manufactured through a fast, simple and cost-effective process.

In accordance with the above objects of the present invention, a pharmaceutical composition for oral administration is provided comprising Valaciclovir in complex with a resin, wherein the ratio of Valaciclovir to the resin is 1:0.8.

According to another embodiment of the present invention, a process for the preparation of a chewable tablet comprising an antiviral agent such as Valaciclovir or pharmaceutical acceptable salt or derivative thereof as an active ingredient in complex with a resin wherein the ratio of Valaciclovir to the resin is 1:0.8 is provided, which comprises the following steps:
- Dry blending of drug: resin in the ratio 1:0.8;
- Kneading the above blend with water;
- Drying of the wet mass at 40°C;
- Milling of the drug-resin complex until particle size gets less than 250µm;
- Dry mixing of the drug-resin complex and the excipients of the internal phase;
- Mixing with the excipients of the external phase;
- Shifting the powder to eliminate any clumps;
- Compressing the resulted mixture into a tablet dosage form.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

As already mentioned the main object of the present invention is to provide a stable pharmaceutical composition of Valaciclovir or pharmaceutical acceptable salt or derivative thereof for oral administration appropriate for children.

Because of physiological and pharmacokinetic differences between the adult and pediatric population there is the need for the development of formulations specifically studied and designed for children. In children gastric emptying time and gastric pH is variable, and there are differences in the surface area of the absorptive sites and gastrointestinal permeability. There are also reported changes in the biliary function depending upon age, body water and adipose tissue, which may lead to differences in drug disposition and elimination. In most cases, a child's dose is calculated based on the body weight, whereas a few cases based on body surface are also in use.

An ideal formulation for children will allow minimal dosage and frequency; will have minimal impact on lifestyle; a minimum of nontoxic excipients and will have convenient, easy and reliable administration.

Children are a very heterogeneous population that includes newborns, infants, toddlers, preschoolers, school-age children and adolescents. Therefore, there is the need to develop formulations appropriate for all the pediatric subpopulations that will use the products.

The present invention proposes a palatable chewable tablet for children above the age of 6 years.

Administration of drugs through oral route is the most common and easiest way to administer a drug. But it is a challenge in children that face troubles in swallowing tablets. Chewable tablets improve the compliance in children as the tablet is broken and chewed in between the teeth before ingestion. The advantages of chewable tablets include palatability, stability, precise dosing, portability and ease of delivery. Moreover, they do not require water, which means that they can be taken at any time and in any place.

The most important problem encountered at the development of Valaciclovir formulations of the present invention was the extremely bitter taste of active ingredient. Taste is an important parameter in administering drugs orally. In paediatric patients, acceptance of a dosage form is primarily dependent on a child's taste preference. Different taste masking technologies have been used to address the problem of patient compliance. Taste masking of water soluble bitter drugs, especially those with a high dose, is difficult to achieve by using sweeteners alone. As a consequence, more efficient techniques such as coating, microencapsulation and granulation have been used in combination with the sweeteners.

The chewable tablets of the present invention may also contain additional formulation excipients such as diluents.

Diluents increase the bulk of a solid pharmaceutical composition, and may make a pharmaceutical dosage form easier for the patient and care giver to handle. Diluents for solid compositions include, for example, dextrates, fructose, mannitol, microcrystalline cellulose, silicified microcrystalline cellulose, sorbitol, starch, pregelatinized starch, sucrose, xylitol, maltose, maltodextrin, maltitol, lactose.

The preferred composition of the present invention comprises preferably Emdex®, i.e. dextrates and Prosolv SMCC® 90, i.e. silicified microcrystalline cellulose.

Dextrates is a purified mixture of saccharides developed from the controlled enzymatic hydrolysis of starch. It is either anhydrous or hydrated. In addition to dextrose, dextrates contains 3-5% w/w maltose and higher polysaccharides. It has a sweet taste and produces a cooling sensation in the mouth.

Silicified microcrystalline cellulose is a mixture of microcrystalline cellulose and colloidal silicon dioxide. It is widely used in formulas where a balance of flow and compaction is required as it has improved compaction properties compared to conventional microcrystalline cellulose. Silicification provides flow that is comparable to doubling the particle size of microcrystalline cellulose in addition to superior compaction.

The chewable tablet proposed by the present invention comprises a complex of Valaciclovir with an ion exchange resin in a specific ratio in order to obtain a palatable and child-friendly product.

Ion exchange Resins (IER) were extensively used in the development of formulations of the present invention as taste masking agents. IERs are solid and suitably insoluble high molecular weight polyelectrolytes that can exchange their mobile ions of equal charge with the surrounding medium. The resulting ion exchange is reversible and stoichiometric with the displacement of one ionic species by another. Research over the last few years has revealed that IERs are equally suitable for drug delivery technologies, including controlled release, transdermal, nasal, topical and taste masking. Being high molecular weight water insoluble polymers, the resins are not absorbed by the body and are therefore inert.

Since most drugs possess ionic sites in their molecule, the resin's charge provides a means to loosely bind such drugs and this complex prevents the drug release in the saliva, thus resulting in taste masking. The nature of the drug resin complex formed is such that the average pH of 6.7 and cation concentration of about 40meq/L in the saliva are not able to break the drug resin complex but it is weak enough to be broken down by hydrochloric acid present in the stomach. Thus, the drug resin complex is absolutely tasteless with no after taste, and at the same time, its bioavailability is not affected.

A critical factor to prepare a Drug-Resin complex (DRC) was the choice of the right IER. Valaciclovir·HCl contains an exchangeable secondary amine moiety i.e. cationic center. Therefore, cation exchange resins are highly recommended for the complex formation. Weak cation acid ion exchange resins such as Indion 204, Indion 214 as well as a strong cation acid resin such as PuroliteC100CaMR were tested in order to form the DRC.

Mediums of different pH were examined in order to achieve the maximum drug loading into the resin. For this reason, the pH of solutions was adjusted at 3, 4, 5, 6, and 7. The drug loaded was evaluated spectrophotometrically. The %w/w unbound drug for pH 3, 4, 5, 6, 7 and water was found to be 79.85% ± 2.19%, 75.24% ± 2.91%, 66.69% ± 2.76%, 61.19% ± 2.61%, 30.91% ± 2.53% and 72.61% ±2.81% respectively. Buffer pH=7 enhanced more the effect of drug loading.

The next step was to examine the effect of drug loading into the resin for buffer pH=7 of different ion strength. For this reason, buffer pH=7 of normality 0.1N, 0.2N, IN were prepared and the drug loaded was evaluated spectrophotometrically as before. Buffer pH=7 0,2N was the most suitable medium as the higher drug loading was reached. (61,60% ± 2.05%).

At this point of studies, the research focused on the type of IER. For this reason, resins of different functional group were examined to reach the higher drug loading. The %w/w unbound drug for Indion 204, Indion 214, Kyron T-134, Kyron T-314, Purolite C115KMR and Purolite C100CaMR was found to be 30.91% ± 1.97%, 44% ± 2.95%, 59.95% ± 2.96%, 87.99% ± 1.67%, 58.42% ± 2.93% and 21.80% ±1.29% respectively. As a result, Purolite C100CaMR and Indion 204 were the most preferred resins that could form hydrogen bonds with the cationic center of Valaciclovir and prevent the release in saliva.

Both Indion 204 and Purolite C100Ca were evaluated for their taste/feel acceptability and drug contamination status. Compositions with Purolite C100Ca did not have the desirable taste; they had a more acidic taste due to its strong nature. Moreover, impurities data of drug-resin complex for Purolite C100Ca showed that Purolite C100Ca degrades the drug to a greater extent than Indion 204. More specifically, increasing the Drug: Purolite C100Ca ratio both guanine and acyclovir follow an increasing trend. This behaviour does not appear to Drug: Indion 204 complex of any ratio.

Consequently, Indion 204 appears as the most suitable resin for the Valaciclovir development. More specifically, Drug: Indion 204 with ratio 1:0.8 is the most appropriate for the development of Valaciclovir due to lower drug degradation effect and optimum taste masking of the composition.

Another important factor that was examined was the quantity of water used for the preparation of the drug-resin complex. The wet granulation method was selected for the DRC preparation as it could perform a stable, easy-to use complex with a neutral taste. It was observed that excessive amounts of water led to a DRC that didn't release the drug even in the stomach environment. DRCs of different Drug: Resin: Water ratio were tested in order to determine the appropriate water quantity. Optimum dissolution profile was achieved when Drug: Resin in the ratio 1:0.8 was granulated with water according to ratio Drug: Resin: Water 1:0.8:0.5.

It is possible to prepare dosage forms of different strength using appropriate quantity of the same composition, thereby limiting the cost of production and minimizing the number, and consequently the cost of clinical studies required for the approval of the product by the authorities.

The following examples illustrate preferred embodiments in accordance with the present invention without limiting the scope or spirit of the invention:

### EXAMPLES

**Table 1: Formulation 1 and 2**

| **Ingredients** | **mg/tab** | |
|---|---|---|
| | **Formulation 1** | **Formulation 2** |
| **Internal Phase** | | |
| Valaciclovir | 250,00 | 250,00 |
| Valaciclovir hydrochloride hydrated | 309,23 | 309,23 |
| Indion 204 | 200,00 | 200,00 |
| Prosolv SMCC 90 | 50,00 | 50,00 |
| Crospovidone | 50,00 | 30,00 |
| Emdex | 40,00 | 60,00 |
| Sucralose | 20,00 | 20,00 |

| **External Phase** | | |
|---|---|---|
| Orange flavour | 2,00 | - |
| Lemon flavour | - | 1,30 |
| Talc | 10,00 | 10,00 |
| Mg stearate | 10,00 | 10,00 |
| **Total solids** | 691,23 | 690,53 |

The process chosen for the preparation of Formulations 1 and 2 comprises the following steps:
- Dry blending of Drug: Indion 204 in the ratio 1:0.8;
- Kneading the above blend with water in the ratio Drug: Indion 204: Water 1:0.8:0.5;
- Drying of the wet mass at 40°C;
- Milling of the Drug-Indion 204 complex until particle size gets less than 250µm;
- Dry mixing of the Drug-Indion 204 complex and the excipients of the internal phase;
- Mixing with the excipients of the external phase;
- Shifting the powder to eliminate any clumps;
- Compressing the obtained mixture into tablet dosage form.

Tablets of Formulation 1 and 2 were tested for their physical characteristics (hardness and disintegration time in Buffer pH=7, 4). The results are presented in table 2 below:

**Table 2: Characteristics of Formulation 1 and 2**

| | **Hardness** | **Disintegration** | **Compression** | **Comments** |
|---|---|---|---|---|
| Formulation 1 | 103 N | 29" | 1ton | Hard tablet |
| Formulation 2 | 60 N | 19" | 1ton | Friable and fragile tablet |

Despite the fact that the disintegration time of Formulation 1 was acceptable (29sec), the tablet was hard enough to be chewed. The taste was also undesirable due to the acidic nature of orange flavour. Thus, in Formulation 2 the orange flavour was replaced by lemon flavour and the diluent (Emdex) was increased in order to prepare a softer tablet. However, Formulation 2 had a quite low disintegration time, the tablet was friable enough to be handled and the taste was unpleasant.

The next step was to replace the flavouring agent with a mixture of strawberry:vanilla 1,5:1,2 The preferred composition of the present invention is presented in table 3 below.

**Table 3: Preferred composition of the present invention**

| **Ingredients** | **mg/tab** |
|---|---|
| **Internal Phase** | |
| Valaciclovir | 250,00 |
| Valaciclovir Hydrochloride hydrated | 309,23 |
| Indion 204 | 200,00 |
| Prosolv SMCC 90 | 50,00 |
| Crospovidone | 30,00 |
| Emdex | 50,00 |
| Sucralose | 20,00 |

| **External Phase** | |
|---|---|
| Strawberry flavour | 1,50 |
| Vanilla flavour | 1,20 |
| Talc | 10,00 |
| Mg stearate | 10,00 |
| **Total solids** | 681,93 |

The preferred composition of the present invention was prepared with the same manufacturing process as Formulation 1 and 2.

Tablets of the preferred composition were also tested for their physical characteristics (hardness and disintegration time in Buffer pH=7, 4) and the results are presented in table 4 below.

**Table 4: Characteristics of the preferred composition of the present invention**

| **Hardness** | **Disintegration** | **Compression** | **Comments** |
|---|---|---|---|
| 79 N | 26" | 1ton | Good appearance |

The preferred tablets of the present invention have desirable taste, acceptable disintegration time and there is no evidence of poor friability of bulk flow. They are easily chewed and there is no bitter after taste.

The development of chewable tablets 500mg/tab and 1000mg/tab followed the weight proportionality rule.

## Claims

1. A chewable tablet comprising Valaciclovir or pharmaceutical acceptable salt or derivative thereof in complex with an ion exchange resin, wherein the ratio of Valaciclovir to the ion exchange resin is 1:0.8.

2. The chewable tablet according to claim 1, wherein the ion exchange resin is a cationic acid ion exchange resin.

3. The chewable tablet according to claim 1, further comprising one or more diluents.

4. The chewable tablet according to claim 3, wherein the diluents are selected from dextrates and silicified microcrystalline cellulose.

5. The chewable tablet according to any preceding claim, wherein it is appropriate for children above the age of 6 years.

6. A process for the preparation of a chewable tablet, as defined in any claim from 1 to 5, comprising the following steps:
- Dry blending of Drug: Ion Exchange Resin in the ratio 1:0.8;
- Kneading the above blend with water in the ratio Drug: Ion Exchange Resin: Water 1:0.8:0.5;
- Drying of the wet mass at 40°C;
- Milling of the Drug Resin Complex until particle size gets less than 250µm;
- Dry mixing of the Drug Resin Complex and the excipients of the internal phase;
- Mixing with the excipients of the external phase;
- Sifting the powder to eliminate any clumps;
- Compressing into tablet dosage form.

7. The process according to claim 6, wherein the ion exchange resin is a cationic acid ion exchange resin.

8. The process according to claim 6, wherein the chewable tablet is appropriate for children above the age of 6 years.

## Patentansprüche

1. Eine Kautablette, die Valaciclovir oder ein pharmazeutisch akzeptables Salz oder Derivat davon in einem Komplex mit einem Ionenaustauscherharz enthält, wobei das Verhältnis von Valaciclovir zu dem Ionenaustauscherharz 1: 0,8 ist

2. Kautablette nach Anspruch 1, wobei das Ionenaustauschharz ein kationisches Säure Ionenaustauschharz ist

3. Kautablette nach Anspruch 1, weiterhin ein oder mehrere Verdünnungsmittel umfassend

4. Kautablette nach Anspruch 3, wobei die Verdünnungsmittel sind aus Dextraten und verkieselter mikrokristalliner Cellulose ausgewählt

5. Kautablette nach einem der vorhergehenden Ansprüche, wobei sie für Kinder über dem Alter von 6 Jahren geeignet ist

6. Verfahren zur Herstellung einer Kautablette nach einem der Ansprüche 1 bis 5, umfassend die folgenden Schritte:
- Arzneimittel:Ionenaustauschharz im Verhältnis 1: 0,8 mischen
- der obigen Mischung mit Wasser im Verhältnis Arzneimittel:Ionenaustauscherharz:Wasser 1: 0,8: 0,5 kneten
- der nassen Masse bei 40°C trocknen
- des Arzneimittel Harz-Komplex, bis die Teilchengröße weniger als 250 um beträgt mahlen
- des Arzneimittels Harz-Komplex und die sonstigen Hilfsstoffe der inneren Phase trockenes mischen
- mit den Hilfsstoffen der äußeren Phase mischen
- des Pulvers sichten, um Klumpen zu entfernen
- in Tablettenform komprimierung

7. Verfahren nach Anspruch 6, wobei das Ionenaustauscherharz ein kationisches Säure Ionenaustauscherharz ist

8. Verfahren nach Anspruch 6, wobei die Kautablette für Kinder über dem Alter von 6 Jahren geeignet ist

## Revendications

1. Un comprimé à mâcher comprenant Valaciclovir ou un sel ou un dérivé pharmaceutiquement acceptable de celui-ci en complexe avec une résine échangeuse d'ions, où le rapport entre le Valaciclovir et la résine échangeuse d'ions est de 1 : 0.8.

2. Le comprimé à mâcher selon la revendication 1, dans lequel la résine échangeuse d'ions est une résine acidique échangeuse de cations.

3. Le comprimé à mâcher selon la revendication 1, comprenant en outre un ou plusieurs diluants.

4. Le comprimé à mâcher selon la revendication 3, dans lequel les diluants sont sélectionnés parmi les dextrates et la cellulose microcristalline silicifiée.

5. Le comprimé à mâcher selon l'une quelconque des revendications précédentes, lequel est approprié aux enfants âgés de plus de 6 ans.

6. Un procédé pour la préparation d'un comprimé à mâcher tel que défini dans l'une quelconque des revendications de 1 à 5, comprenant les étapes suivantes :
- Mélange à sec des Médicament : Résine échangeuse d'ions dans le rapport 1 : 0.8 ;
- malaxage du mélange ci-dessus avec de l'eau dans un rapport Médicament : Résine échangeuse d'ions : Eau de 1 : 0.8 : 0.5 ;
- séchage de la masse mouillée à 40°C ;
- mouture du Complexe Médicament-Résine jusqu'à ce que la taille des particules devienne inférieure à 250 µm ;
- mélange à sec du Complexe Médicament-Résine avec les excipients de la phase interne ;
- mélange avec les excipients de la phase externe ;
- tamisage de la poudre pour éliminer les agrégats ;
- compressage sous forme de comprimé.

7. Le procédé selon la revendication 6, dans lequel la résine échangeuse d'ions est une résine acidique échangeuse de cations.

8. Le procédé selon la revendication 6, dans lequel le comprimé à mâcher est approprié pour les enfants âgés de plus de 6 ans.
